(19) Europäisches Patentamt
European Patent Office
Office européen des brevets

(11) EP 0 572 870 B1

(12) EUROPÄISCHE PATENTSCHRIFT

(45) Veröffentlichungstag und Bekanntmachung des Hinweises auf die Patenterteilung:
**17.07.1996 Patentblatt 1996/29**

(51) Int. Cl.$^6$: **C07C 69/96**, C07C 68/06

(21) Anmeldenummer: **93108199.6**

(22) Anmeldetag: **19.05.1993**

# (54) Verfahren zur Herstellung von organischen Carbonaten mit mindestens einer aromatischen Estergruppe

Process for the production of organic carbonates with at least one aromatic ester group

Procédé de fabrication de carbonates organiques avec au moins un groupe ester aromatique

(84) Benannte Vertragsstaaten:
**BE DE ES FR GB IT NL**

(30) Priorität: **01.06.1992 DE 4218061**

(43) Veröffentlichungstag der Anmeldung:
**08.12.1993 Patentblatt 1993/49**

(73) Patentinhaber: **BAYER AG**
**51368 Leverkusen (DE)**

(72) Erfinder:
- **Wagner, Paul, Dr.**
  **W-4000 Düsseldorf (DE)**
- **Schön, Norbert, Dr.**
  **W-4150 Krefeld (DE)**
- **Buysch, Hans-Josef, Dr.**
  **W-4150 Krefeld (DE)**

(56) Entgegenhaltungen:
**EP-A- 0 461 274**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 572 870 B1

Printed by Rank Xerox (UK) Business Services
2.13.0/3.4

## Beschreibung

Die Erfindung betrifft ein kontinuierliches Verfahren zur Herstellung von Arylcarbonaten aus mindestens eine aliphatische Estergruppe enthaltenden Carbonaten einerseits und Phenolen oder Alkylarylcarbonaten andererseits durch katalysierte Umesterung in einem kolonnenartigen Reaktor unter mehrfacher Rückführung der Reaktionsprodukte in denselben Reaktor und Zwischenlagerung der Produktströme in geeigneten Behältern.

Die Herstellung von aromatischen und aliphatisch-aromatischen Kohlensäureestern (Carbonaten) durch Umesterung, ausgehend von aliphatischen Kohlensäureestern und Phenolen, ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion jedoch im umgekehrten Sinne in Richtung aromatischer Carbonate durchzuführen, ist es notwendig, das sehr ungünstig liegende Gleichgewicht effektiv zu verschieben, wobei nicht nur sehr aktive Katalysatoren, sondern auch eine günstige Verfahrensweise zur Anwendung gelangen müssen.

Zur Umesterung von aliphatischen Kohlensäureestern mit Phenolen sind eine Vielzahl von effektiven Katalysatoren, wie beispielsweise Alkalihydroxide, Lewissäure-Katalysatoren aus der Gruppe der Metallhalogenide (DE-OS 25 28 412 und DE-OS 25 52 907), Organozinnverbindungen (EP-879, EP-880, DE-OS 34 45 552, EP-338 760), Bleiverbindungen (JP-57/176 932), Lewissäure/Protonensäure-Katalysatoren (DE-OS 34 45 553), empfohlen worden. In den bekannten Verfahren wird die Umesterung in einem chargenweise betriebenen Reaktor drucklos oder unter Druck, gegebenenfalls mit einer zusätzlichen Trennkolonne, durchgeführt. Dabei werden auch mit den aktivsten Katalysatoren Reaktionszeiten von vielen Stunden bis zum Erreichen auch nur mittlerer Umsätze von ungefähr 50 % an Phenol benötigt. So werden bei der chargenweise betriebenen Umesterung von Phenol mit Diethylcarbonat bei 180°C unter Verwendung verschiedener Organozinnverbindungen, wie sie in DE-OS 34 45 552 beschrieben werden, Ausbeuten an Diphenylcarbonat in einer Größenordnung von mehr als 20 % erst nach ca. 24-stündiger Reaktionszeit erreicht; bei der chargenweise betriebenen Umesterung von Phenol und Dimethylcarbonat mit Hilfe von Organozinnkatalysatoren, wie sie in EP-879 beschrieben sind, beträgt der Phenolumsatz nach 30 Stunden 34 % des theoretischen Wertes.

Das bedeutet, daß aufgrund der ungünstigen thermodynamischen Voraussetzungen die beschriebenen Umesterungsreaktionen in Kesseln oder Druckautoklaven auch bei Verwendung sehr aktiver Katalysatorsysteme im Sinne eines technischen Prozesses nur sehr unvorteilhaft durchführbar sind, da sehr schlechte Raum-Zeit-Ausbeuten und hohe Verweilzeiten bei hohen Reaktionstemperaturen erforderlich sind, wobei wegen der unvollständigen Umesterung zusätzlich ein hoher Destillationsaufwand aufgebracht werden muß, der weitere Energie erforderlich macht.

Solche Verfahrensweisen sind auch deshalb besonders unvorteilhaft, da selbst mit sehr selektiven Umesterungskatalysatoren bei den hohen Temperaturen und langen Verweilzeiten von vielen Stunden ein merklicher Anteil an Nebenreaktionen auftritt, beispielsweise die Etherbildung und die Abspaltung von Kohlendioxid.

Es wurde daher versucht, das Reaktionsgleichgewicht durch Adsorption des bei der Umesterung entstehenden Alkohols an Molekularsieben möglichst schnell in Richtung der gewünschten Produkte zu verschieben (DE-OS 33 08 921). Aus der Beschreibung dieser Verfahrensweise zeigt sich, daß zur Adsorption des Reaktionsalkohols eine große Menge an Molekularsieb benötigt wird, die die Menge an freiwerdendem Alkohol weit überschreitet. Weiterhin müssen die eingesetzten Molekularsiebe schon nach kurzer Zeit regeneriert werden, und die Umwandlungsrate zu den Alkylarylcarbonat-Zwischenprodukten ist relativ gering. Auch dieses Verfahren erscheint deshalb als technisch nicht vorteilhaft anwendbar.

Es ist bekannt, Gleichgewichtsreaktionen, insbesondere Veresterungen und Umesterungen in Kolonnen durchzuführen, sie auf diese Weise zu beschleunigen und in Richtung auf die gewünschten Produkte zu verschieben (Chem.-Ing.-Techn. 49, 151 (1977); DE-OS 38 09 417; Chem.-Ing.-Techn. 62, 226 (1990); Ullmanns Encyclopädie der techn. Chemie, 4.Aufl., Bd.3, S. 375ff. (1973)). Ein in WO 91/09832 ≙ EP 0 461 274 beschriebener kontinuierlicher Umesterungsprozess zur Herstellung von aromatischen Carbonaten, bei dem die Reaktion mehrstufig in hintereinander geschalteten Kolonnen durchgeführt wird, ist eine optimierte Ausführung dieses Reaktionsprinzips. Dabei werden in den beschriebenen Kolonnen Phenole mit Dialkylcarbonaten umgesetzt, wobei über Kopf die tiefersiedenden Reaktionsprodukte, nämlich aliphatische Alkohole zusammen mit nicht umgesetzten Dialkylcarbonaten und am Fuß der Kolonne die schwersiedenden Reaktionsprodukte, nämlich Alkylarylcarbonate und gegebenenfalls Diarylcarbonate entnommen werden. In einer weiteren, nachgeschalteten Kolonne werden dann die schon gebildeten Alkylarylcarbonate zu den gewünschten Diarylcarbonat-Endprodukten umgesetzt. Die dabei als Koppelprodukte entstehenden Dialkylcarbonate und gegebenenfalls Alkohole und die noch nicht umgesetzten Phenole werden am oberen Ende der Kolonne entnommen und teilweise oder vollständig in die erste Kolonne recyclisiert. Wie aber aus den Ausführungsbeispielen und den beschriebenen Verfahrensvarianten hervorgeht, sind die Umsätze der Phenole und Dialkylcarbonate in der ersten Umesterungskolonne selbst unter günstigen Bedingungen, wie hohen Temperaturen und Drücken und großen Überschüssen an Dialkylcarbonaten von 100 bis 300 %, auf geringe Werte begrenzt, d.h., auch in günstigen Fällen, wie im Beispiel 10 von WO 91/09832 enthält der Sumpf nur ca. 15 Gew.-% Umesterungsprodukte, im wesentlichen Methylphenylcarbonat.

Das bedeutet, daß in der nachfolgenden zweiten Kolonne nur ein geringer Teil des Eduktstroms, nämlich das schon gebildete Alkylphenylcarbonat zum Diarylcarbonatendprodukt umgesetzt werden kann mit der unvorteilhaften Konsequenz, daß der überwiegende Rest, der im wesentlichen aus dem Phenol besteht, destillativ über Kopf entfernt und nach Kondensation in die erste Kolonne zurückgeführt werden muß.

Dafür muß ein unverhältnismäßig hoher Destillations- und Energieaufwand aufgebracht werden. Die zu benutzenden Kolonnen müssen bei vorgegebener Produktionsmenge pro Zeiteinheit große Volumina besitzen und erfordern hohe Investitionskosten, insbesondere erfordert das Abdestillieren der großen Phenol-, Dialkylcarbonat- und gegebenenfalls Alkoholmengen, das bevorzugt im Vakuum ausgeführt wird, wobei große Gasmengen entstehen, eine sehr große und damit teure und schwierig zu betreibende Kolonne. Außerdem ist die Regelung mehrerer kontinuierlich betriebener und hintereinander geschalteter Kolonnen, deren Produktströme jeweils voneinander abhängig sind, aufwendig und schwierig.

Die nach WO 91/09832 erzielte, wenn auch unzureichende Verbesserung überrascht jedoch nicht, da es allgemein bekannt ist, daß Umesterungen in Kolonnen häufig rascher ablaufen, was ja auch im vorliegenden Fall beobachtet wird. Allerdings sind die erhaltenen Umsätze sehr gering, und man ist offenbar davon ausgegangen, daß sie bei der ungünstigen Gleichgewichtslage ($K \cong 10^{-3}$) auch unter optimalen Bedingungen, d.h. hohen Temperaturen und Drücken, praktisch nicht, d.h. nur unter sehr großem Aufwand, weiter erhöht werden können. Eine solch ungünstige Gleichgewichtslage bedeutet, daß im Gleichgewicht nur ca. 2 bis 3 Gew.-% Produkt vorliegen und der Umsatz nur dann weiter erhöht werden kann, wenn eine Produktkomponente, hier der Reaktionsalkohol entfernt wird und sich das Reaktionssystem erneut ins Gleichgewicht setzen kann. Dieser Vorgang müßte viele Male wiederholt werden. Bei einer langsamen Einstellung des Gleichgewichts, wie im vorliegenden Fall, müßte man dazu sehr lange, vielbödige Kolonnen benutzen, die nur mit geringer Belastung und geringer Raum-Zeit-Ausbeute betrieben werden könnten. Solche Bedingungen hat man in WO 91/09832 offensichtlich als nicht realisierbar angesehen.

Das Ziel einer Verbesserung des Umesterungsprozesses muß es also sein, mit einem geeigneten Reaktionsapparat unter geeigneten Bedingungen in kontinuierlicher, möglichst einfacher Fahrweise größere Phenol-Umsätze bzw. kleinere Restgehalte an Phenol im Sumpfprodukt als bisher zu realisieren.

Dieses Ziel läßt sich nun durch das erfindungsgemäße, quasi-kontinuierlich arbeitende katalysierte Umesterungsverfahren erreichen. Dabei wird ein kolonnenartiger Reaktor vom Produktstrom mehrfach durchlaufen, wodurch überraschenderweise eine gewünschte Zusammensetzung, die erheblich mehr Umesterungsprodukte bzw. weniger Phenol enthält, erhalten wird. Das nach einem Durchlauf durch die Kolonne erhaltene Produkt muß im Gegensatz zum oben angeführten Verfahren nicht destillativ aufkonzentriert werden, sondern wird in der beim vorangegangenen Durchlauf erhaltenen flüssigen Form in den oberen Teil der Kolonne zurückgepumpt, so daß Destillationsaufwand entfällt. Um bei mehrfacher Nutzung einer Kolonne eine kontinuierliche Arbeitsweise realisieren zu können, werden die Produktströme in einem geeigneten Behälter mit zwei oder mehreren abgeteilten Räumen zwischengelagert und jeweils aus einem dieser Räume taktweise in die Kolonne zurückgeführt. Für eine solche Verfahrensweise ist nur ein geringer apparativer Aufwand, nämlich im einfachsten Fall eine Kolonne, ein geeigneter, billiger Zwischenlagerraum und außerdem geringer regelungstechnischer Aufwand notwendig, was letztlich einen geringen Investitionsaufwand bedeutet. Da die Edukt- und Produktströme taktweise, in zeitlich festgelegten Cyclen oder z.B. über vorgegebene Produktzusammensetzungen oder Temperaturen geregelt werden können, wobei die Zusammensetzung eines Produktstroms eine einfache Regelgröße darstellt, ist der regelungstechnische Aufwand des Verfahrens sehr gering.

Beim Benutzen mehrerer Kolonnen, wie in WO 91/09832 beschrieben, müssen für jede Kolonne die zugehörigen Nebenapparate und Regelungseinheiten aufgebaut werden, wobei die Regelung viel mehr Aufwand als beim erfindungsgemäßen Verfahren erfordert, da die einzelnen Regelungsgrößen in mehrfacher Hinsicht voneinander abhängig sind. Da in der erfindungsgemäßen Verfahrensausführung eine destillative Abtrennung der nicht umgesetzten Edukte erst bei viel höheren Umsätzen erfolgen kann, wird deutlich weniger Reaktionsraum und auch weniger Energie benötigt. Besonders angesichts der Tatsache, daß die Gleichgewichtslage für die gewünschten Arylcarbonate ($K \cong 10^{-3}$) äußert ungünstig ist, muß es im höchsten Maße überraschen, daß nach dem erfindungsgemäßen Verfahren so bedeutende Umsatzerhöhungen gegenüber dem Stand der Technik möglich sind.

Die Erfindung betrifft demnach ein Verfahren zur Herstellung eines organischen Carbonats mit mindestens einer aromatischen Estergruppe der Formel

$$R^1\text{-OCOO-}R^2 \qquad (I),$$

in der

$R^2$ Phenyl oder Naphthyl sowie ein- bis dreifach durch geradkettiges oder verzweigtes $C_1$-$C_4$-Alkyl, geradkettiges oder verzweigtes $C_1$-$C_4$-Alkoxy, Cyano und/oder Halogen substituiertes Phenyl bzw. Naphthyl bedeutet und

$R^1$ unabhängig von $R^2$ den Bedeutungsumfang von $R^2$ annehmen oder geradkettiges oder verzweigtes $C_1$-$C_6$-Alkyl bedeuten kann,

durch katalysierte Umsetzung von je 0,1-10 mol, bevorzugt 0,2-5 mol, besonders bevorzugt 0,5-3 mol, eines organischen Carbonats mit mindestens einer aliphatischen Estergruppe der Formel

$$R^1\text{-OCOO-}R^3 \qquad (II),$$

in der

$R^3$ geradkettiges oder verzweigtes $C_1$-$C_6$-Alkyl bedeutet und
$R^1$ den obigen Bedeutungsumfang hat,

mit je 1 mol einer phenolischen Verbindung der Formel

$$R^2\text{-OX} \qquad (III),$$

in der

$R^2$ den obigen Bedeutungsumfang hat und
X für Wasserstoff oder für -COO-$C_1$-$C_6$-Alkyl mit geradkettiger oder verzweigter Alkylgruppe steht,

in Gegenwart eines an sich bekannten Umesterungskatalysators bei 60-320°C in einem kolonnenartigen Reaktor, wobei das mindestens eine aromatische Estergruppe enthaltende organische Carbonat im unteren Teil der Kolonne und die als Reaktionsprodukt mitentstehende alkoholische Verbindung der Formel

$$R^3\text{-OX} \qquad (IV),$$

in der

X und $R^3$ die genannte Bedeutung haben,

im oberen Teil der Kolonne entnommen werden, das dadurch gekennzeichnet ist, daß das im unteren Teil der Kolonne flüssig entnommene Sumpfprodukt, das mindestens eine aromatische Estergruppe enthaltendes Carbonat, noch nicht umgesetztes Phenol und gegebenenfalls geringe Mengen des mindestens eine aliphatische Estergruppe enthaltenden Carbonats enthält, 1 bis 10, bevorzugt 1 bis 5, weiteren Durchläufen durch denselben Reaktor unter Zwischenlagerung des Sumpfprodukts unterworfen wird, wobei bei den zuletzt angesetzten 1-4 Durchläufen der weitere Zusatz des organischen Carbonats mit mindestens einer aliphatischen Estergruppe entfallen kann.

Die Umesterung im erfindungsgemäßen Verfahren umfaßt mehrere Reaktionen, wie die folgenden Gleichungen in verallgemeinerter Form zeigen:

Alkyl-OCOO-Alkyl + Aryl-OH → Aryl-OCOO-Alkyl + Alkyl-OH (Gleichung 1)

Aryl-OCOO-Alkyl + Aryl-OH → Aryl-OCOO-Aryl + Alkyl-OH (Gleichung 2)

2 Aryl-OCOO-Alkyl → Aryl-OCOO-Aryl + Alkyl-OCOO-Alkyl (Gleichung 3)

Bei der Bildung eines Diarylcarbonats erfolgt die Umesterung von den aliphatischen zu den aromatischen Estergruppen in zwei Stufen, wobei ein Alkylarylcarbonat im Sinne der Gleichung 1 als Produkt der ersten Umesterungsstufe durchlaufen wird.

Die Gleichung 3 zeigt ferner eine Disproportionierungsreaktion, in welcher aus einem gemischten Alkylarylcarbonat sowohl das symmetrische Dialkylcarbonat als auch das gewünschte symmetrische Diarylcarbonat entstehen. Es ist ferner möglich, das Alkylarylcarbonat als das gewünschte Reaktionsprodukt zu erhalten, also nur die erste Umesterungsstufe zu betreiben. Noch weiterhin ist es möglich, durch Einsatz von Gemischen verschiedener Phenole auch unsymmetrische Diarylcarbonate zu erhalten.

Zum Einsatz gelangen Dialkylcarbonate mit gleichen oder verschiedenen aliphatischen Estergruppen mit geradkettigem oder verzweigtem $C_1$-$C_6$-Alkyl. Solche Dialkylcarbonate sind dem Fachmann bekannt und können nach bekannten Verfahren hergestellt werden. Aus Gründen der Verbilligung wird man im allgemeinen von symmetrischen Dialkylcarbonaten ausgehen.

Geradkettiges oder verzweigtes $C_1$-$C_6$-Alkyl ist beispielsweise Methyl, Ethyl, Propyl, Isopropyl, Butyl, Isobutyl, Pentyl oder Hexyl.

Geradkettiges oder verzweigtes $C_1$-$C_4$-Alkoxy ist beispielsweise Methoxy, Ethoxy, Propoxy, Isopropoxy, Butoxy oder Isobutoxy.

Halogen ist beispielsweise Fluor, Chlor oder Brom, bevorzugt Fluor oder Chlor, besonders bevorzugt Chlor.

Die aromatische Estergruppe kann von einem Phenol oder einem Naphthol, bevorzugt von einem Phenol abgeleitet sein und in der angegebenen Weise ein- bis dreifach, bevorzugt ein- oder zweifach, besonders bevorzugt einfach substituiert sein. Der Cyano-Substituent tritt in der Regel nur einfach als Substituent auf. Ganz besondere Bedeutung hat das erfindungsgemäße Verfahren für die Umesterung mit Hilfe von nicht substituiertem Phenol.

Erfindungsgemäß einsetzbare Phenole, die unter die Formel (III) fallen, wenn X für Wasserstoff steht, sind beispielsweise nicht substituiertes Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol und 2-Naphthol.

Bevorzugt einsetzbare Phenole sind demnach allgemein solche der Formel

$$R^{12}\text{-OH} \qquad (V),$$

in der

$R^{12}$ Phenyl oder einfach durch $C_1$-$C_4$-Alkyl, $C_1$-$C_4$-Alkoxy oder Chlor substituiertes Phenyl bedeutet.

Hierunter ist das nicht substituierte Phenol besonders bevorzugt.

Als organische Carbonate mit mindestens einer aliphatischen Estergruppe werden bevorzugt symmetrische Dialkylcarbonate der Formel

$$R^3\text{-OCOO-}R^3 \qquad (VI),$$

in der

$R^3$ die angegebene Bedeutung hat,

eingesetzt.

Erfindungsgemäß einsetzbare Dialkylcarbonate sind beispielsweise Dimethylcarbonat, Diethylcarbonat, Dipropylcarbonat, Dibutylcarbonat und Dihexylcarbonat. Bevorzugt einsetzbare Dialkylcarbonate sind Dimethyl- und Diethylcarbonat.

Erfindungsgemäß herstellbare Diarylcarbonate sind beispielsweise Diphenylcarbonat, die symmetrisch und unsymmetrisch substituierten isomeren Biskresylcarbonate, die symmetrisch und unsymmetrisch substituierten isomeren Bis(chlorphenyl)-carbonate, die symmetrisch und unsymmetrisch substituierten isomeren Bis(methoxyphenyl)-carbonate, die symmetrisch und unsymmetrisch substituierten isomeren Bis(ethoxyphenyl)-carbonate, Bis(2,6-dimethylphenyl)-carbonat, Bis(2,4-dimethylphenyl)-carbonat, Di-1-naphthyl-carbonat und Di-2-naphthyl-carbonat, außerdem weitere unsymmetrisch substituierte Diarylcarbonate, beispielsweise die isomeren Kresyl-phenyl-carbonate, die isomeren (Chlorphenyl)phenyl-carbonate, die isomeren (Methoxyphenyl)phenyl-carbonate, die isomeren Naphthyl-phenyl-carbonate und 1-Naphthyl-2-naphthyl-carbonat.

Bevorzugte erfindungsgemäß herstellbare Diarylcarbonate sind solche der Formeln

$$R^{15}\text{-OCOO-}R^{12} \qquad (VII) \text{ bzw.}$$

$$R^{12}\text{-OCOO-}R^{12} \qquad (VIII),$$

in denen

$R^{12}$ und $R^{15}$ unabhängig voneinander den für $R^{12}$ weiter oben angegebenen Bedeutungsumfang haben.

Besonders bevorzugt herstellbares Diarylcarbonat ist Diphenylcarbonat.

Erfindungsgemäß herstellbare Alkylarylcarbonate sind beispielsweise $C_1$-$C_6$-Alkyl-phenyl-carbonate, wie Methyl-phenyl-carbonat, Ethyl-phenyl-carbonat, Propyl-phenyl-carbonat, Butyl-phenyl-carbonat und Hexyl-phenyl-carbonat, $C_1$-$C_6$-Alkyl(o-, m-, p-kresyl)-carbonate, wie Methyl-(o-kresyl)-carbonat, Methyl-(p-kresyl)-carbonat, Ethyl-(o-kresyl)-carbonat, Ethyl-(p-kresyl)-carbonat, $C_1$-$C_6$-Alkyl-(o-, m-, p-chlorphenyl)-carbonate, wie Methyl- oder Ethyl-(p-chlorphenyl)-carbonat und analoge Verbindungen. Besonders bevorzugt herstellbare Alkylaryl-carbonate sind Methyl-phenyl-carbonat und Ethyl-phenyl-carbonat.

Erfindungsgemäß wird der Sumpfablauf nach dem 1. Durchlauf durch die Kolonne mindestens einem weiteren Durchlauf unterworfen, im allgemeinen 1 bis 10 weiteren Durchläufen, bevorzugt 1 bis 5 weiteren Durchläufen. Der entnommene Sumpfablauf verbleibt in der flüssigen Phase; eine destillative Aufkonzentration entfällt also. Der Sumpfablauf bleibt annähernd bei der Temperatur, mit der er entnommen wurde, so daß die fühlbare Wärme im System verbleibt.

In einer vom Apparateeinsatz her besonders günstigen Form wird der Sumpfablauf zu einem oder bis zu 10 weiteren Durchläufen wieder in die selbe Kolonne zurückgeführt, wozu eine Zwischenlagerung vorgenommen wird (kontinuierliches Semi-Batch-Verfahren). Diese mehrphasige Fahrweise wird anhand von Fig. 1 erläutert.

In einer ersten Phase wird ein Phenol über die Leitung (1), gegebenenfalls zusammen mit einem Katalysator, am Kopf der Kolonne (A) eingespeist und diesem vom Fuß der Kolonne aus ein Dialkylcarbonat über (7) gasförmig entgegengeführt. Der im Gegenstrom geführte Dialkylcarbonatstrom kann im untergeordneten Maß den zugrundeliegenden Alkohol, bevorzugt <5 Gew.-% und besonders bevorzugt <1 Gew.-%, bezogen auf den Gesamtdialkylcarbonatstrom, enthalten. Ganz besonders bevorzugt ist jedoch eine alkoholfreie Dialkylcarbonatdosierung bei (7). Die leichtsiedenden Produkte werden am Kopf der Kolonne über die Leitung (8) und die schwer siedenden Reaktionsprodukte am Fuß der Kolonne entnommen und über (2) oder (3) in einem Raum (B.1) des Zwischenlagers (B) geleitet, bis dieser gefüllt ist. Das Zwischenlager kann auch aus mehreren Tanks bestehen, von denen in Fig. 1 nur 2, nämlich (B.1) und (B.2) dargestellt sind.

Dann wird in der zweiten Phase die Phenolzuspeisung (1) unterbrochen und das zwischengelagerte Produkt über (4) an das obere Ende der Kolonne zurückgeführt, wobei weiterhin Dialkylcarbonat über (7) im Gegenstrom dosiert werden kann. Das während dieser Zeit am Kolonnenfuß austretende Produkt wird nun in einem zweiten Zwischenlagertank (B.2) aufgefangen, bis dieser gefüllt ist. Die in den Lagertanks (B.1) oder (B.2) enthaltenen Zwischenproduktgemische können nun wechselweise solange im Kreis unter Dialkylcarbonatgegenstrom gefahren werden, bis ein gewünschter Umesterungsgrad bzw. Phenolverbrauch, der >5 Gew.-%, bevorzugt >20 Gew.-% und besonders bevorzugt >30 Gew.-% an Phenol, bezogen auf das Gesamtumesterungsgemisch, betragen kann, erreicht ist. Dann wird in einer dritten Phase das Zwischenproduktgemisch über (4) in gleicher Weise wie oben, jedoch ohne Dialkylcarbonatgegenstrom solange im Kreis geführt, bis das vorhandene Alkylarylcarbonat nahezu vollständig zum gewünschten Diarylcarbonat umgesetzt ist. In einer vierten und dann letzten Phase ist es gegebenenfalls möglich, noch eventuell vorhandenes Phenol oder Alkylarylcarbonat destillativ über Kopf und zwar ebenfalls über (8) zu entfernen, wobei das Produktgemisch weiterhin in beschriebener Weise im Kreis durch die Kolonne gefahren wird und entweder die Temperatur heraufgesetzt oder der Druck in der Kolonne abgesenkt wird. Am Ende dieser Schritte wird das nun schon nahezu reine Produkt, das gegebenenfalls noch den Katalysator enthalten kann, am Fuß der Kolonne über (10) ausgespeist und gegebenenfalls einer Reinigungsstufe zugeführt. Die Größe der zu verwendenden Zwischenlagerbehälter richtet sich nach der angestrebten Produktionsmenge und der für die Umsetzung benötigten Zeit.

(5) bedeutet einen Rückfluß auf die Kolonne; (6) ist der Umlauf durch den Sumpfaufkocher. Bei (9) kann bei Bedarf Katalysator zudosiert werden. Die Wärmeaustauscher wurden nicht numeriert; sie sind dem Fachmann ebenso wie nicht eingezeichnete Pumpen und Ventile geläufig.

Weiterhin können mit der erfindungsgemäßen Verfahrensweise natürlich auch nur ein Teil der beschriebenen Reaktionsphasen durchgeführt werden, beispielsweise ein Alkylarylcarbonat entweder mit einem Phenol im Sinne einer Umesterung nach Gl.2 oder mit sich selbst im Sinne einer Disproportionsierungsreaktion nach Gl.3 zu Diarylcarbonaten umgesetzt werden, wobei, wie in Phase 4 beschrieben, kein Dialkylcarbonat entgegengeschickt wird. Ebenso ist es möglich, nur die Phasen 1 und 2 zu realisieren, d.h. die Umsetzung von Phenolen mit Dialkylcarbonaten.

Der einzusetzende kolonnenartige Reaktor stellt im einfachsten Fall ein isotherm beheiztes, mit üblichen, für Destillationen zu verwendenden Füllkörpern oder Packungen gefülltes Rohr dar, auf das am Kopf das Phenol, das gegebenenfalls den Katalysator gelöst enthalten kann, aufgegeben wird. Diesem Gemisch wird von unten in Dampfform das einzusetzende Dialkylcarbonat entgegengeschickt. Am Kopf der Kolonne werden die leichtsiedenden Produkte, die Reaktionsalkohole und Dialkylcarbonate, am unteren Ende der Kolonne die schwersiedenden Produkte, Alkylphenylcarbonat, Phenole und gegebenenfalls Diarylcarbonate kontinuierlich entnommen und in ein Zwischenlager mit zwei oder mehreren abgeteilten Räumen geleitet.

Die Kolonne kann am unteren Ende ein bei höheren Temperaturen arbeitendes Abtriebsteil enthalten, in dem eine weitgehende bis vollständige Separierung des eindosierten Dialkylcarbonats von der herabrieselnden Flüssigphase erfolgt, wobei es wieder dampfförmig in den Umesterungsbereich der Kolonne geführt wird. Weiterhin kann die Kolonne am oberen Teil ein Verstarkerteil beinhalten, das mitverdampftes Phenol oder Alkylphenylcarbonat von den leichtsiedenden Reaktionsalkoholen, bzw. Dialkylcarbonaten abtrennt und flüssig in den Umesterungsteil der Kolonne zurückführt.

Bei größeren Kolonnen ist es zweckmäßig, die für die Reaktion notwendige Energie nicht über eine Mantelbeheizung, sondern sowohl mit dem eingesetzten Phenol, als auch mit dem gasförmig eindosierten Dialkylcarbonat einzubringen. Die Aufheizenergie für das zu dosierende Phenol und die Verdampfungsenergie für das Dialkylcarbonat kann über separate oder in der Säule integrierte Verdampfer aufgebracht werden. Im Mittelteil der Kolonne, in der der größte Teil der Umesterung abläuft, kann eine Erweiterung des Kolonnendurchmessers bis auf das Vierfache der restlichen Teile von Vorteil sein. Weiterhin können in die Säule zum Ausgleich von Reaktionswärmen innen- oder außenliegende

Wärmetauscher eingebaut werden. Die Kolonne kann über die gesamte Lange entweder gleiche Temperatur oder einen Temperaturgradienten aufweisen. Die Auslegung des Umesterungs-, des Abtriebs- und des Verstärkerteils kann vom Fachmann vorgenommen werden.

Die zu verwendenden Füllkörper bzw. geordnete Packungen sind die für Destillationen an sich üblichen, wie sie beispielsweise in Ullmann's Encyclopädie der Technischen Chemie, 4. Aufl., Bd. 2, S. 528 ff. oder in den Firmenschriften der in Frage kommenden Apparatebaufirmen beschrieben sind. Beispielsweise seien genannt: Raschig- oder Pallringe, Berl-, Intalox- oder Torussättel, Interpackkörper aus verschiedenen Materialien, wie Glas, Steinzeug, Porzellan, Edelstahl, Kunststofff, die insbesondere bei der Verwendung von Metall, gewebe- oder maschenartig verarbeitet sein können. Bevorzugt sind Füllkörper und geordnete Packungen, die eine große Oberfläche, eine gute Benetzung sowie ausreichende Verweilzeit der Flüssigphase aufweisen.

Diese sind beispielsweise Pall- und Novaloxringe, Berlsättel, BX-Packungen, Montz-Pak, Mellapak, Melladur, Kerapak und CY-Packungen.

Für das erfindungsgemäße Verfahren sind jedoch nicht nur Füllkörperkolonnen geeignet, sondern bevorzugt solche mit festen Einbauten. Geeignet sind allgemein Bodenkolonnen, z.B. solche mit Sieb-, Glocken-, Ventil-, Tunnel- und Zentrifugalböden, die weiterum in unterschiedlichen Ausführungen vorliegen können. Hierunter sind solche mit Glokken- bzw. Ventilböden mit hohen Verweilzeiten bei gutem Stoffaustausch, beispielsweise solche mit hohen Überlaufwehren, besonders bevorzugt.

Die Kolonne wird so betrieben, daß man in die obere Hälfte, bevorzugt in das obere Drittel, das Phenol oder ein Alkylarylcarbonat, das gegebenenfalls den Katalysator gelöst und suspendiert enthält, bevorzugt mit der an dieser Stelle der Kolonne herrschenden Temperatur, flüssig eindosiert. Alternativ kann der Katalysator auch separat im Reaktionsalkohol oder in einem systemfremden, geeigneten, inerten Lösungsmittel gelöst eingebracht werden. Im Falle der Verwendung heterogener Katalysatoren können diese im Gemisch mit den genannten Füllkörpern, in geeigneter Form anstelle von Füllkörpern oder als Schüttung auf eingebauten Kolonnenböden eingesetzt werden. In die untere Hälfte der Kolonne, bevorzugt oberhalb einer gegebenenfalls vorhandenen Abtriebszone, wird das Dialkylcarbonat, in der Regel in Dampfform, eindosiert. Es kann außerdem zweckmäßig sein, zusätzlich an einer Stelle oberhalb der Dialkylcarbonatdosierung, Gemische von Dialkylcarbonaten und den Reaktionsalkoholen, deren Zusammensetzung der der Dampfphase an dieser Stelle der Kolonne entspricht, aufzugeben.

Eine weitere zweckmäßige Verfahrensweise besteht darin, an einer oder mehreren Stellen der Kolonne die Gasphase zu entnehmen und durch frisches gasförmiges Dialkylcarbonat zu ersetzen. Nach Passieren der Umesterungszone wird der Reaktionsalkohol, bevorzugt nach Durchlaufen einer Verstärkerzone, am Kopf der Kolonne entnommen. Er enthält im allgemeinen noch überschüssiges oder nicht umgesetztes Dialkylcarbonat. Nach einmaligem Passieren der Umesterungszone und bevorzugt eines Abtriebsteils tritt am Fuße der Kolonne ein Gemisch aus Alkylarylcarbonat mit überschüssigem oder nicht umgesetztem Phenol, gegebenenfalls geringen Mengen schon gebildetem Diarylcarbonat und gegebenenfalls löslichen Katalysatoren aus.

Dieses Gemisch wird in einem Raum eines, mehrere abgeteilte Räume enthaltenden Behälters aufgefangen, bis dieser gefüllt ist, wobei es bevorzugt ungefähr auf der innerhalb der Kolonne in der Flüssigphase herrschenden Temperatur gehalten wird, was durch eine Isolierung oder eine separate Heizung bewerkstelligt werden kann. Dann wird es über die Leitung (4) in die Kolonne zurückgespeist, wobei die Eindosierung von frischem Phenol oder Alkylarylcarbonat über (1) unterbrochen wird. Das während dieser Zeit am Kolonnenfüß anfallende Produktgemisch wird in einem zweiten separaten Raum des Behälters aufgefangen. Auf diese Weise können die oben beschriebenen Reaktionsphasen nacheinander durchgeführt werden. Es ist möglich und in vielen Fällen zweckmäßig, die Temperatur und gegebenenfalls den Druck mit fortschreitender Reaktion, besonders während der dritten Phase des Verfahrens anzuheben.

Das Molverhältnis der in die Kolonne eingesetzten Edukte variiert von 0,1-10 mol, bevorzugt von 0,2-5 mol und besonders bevorzugt von 0,5-3 mol Dialkylcarbonat pro Mol eingesetztem Phenol.

Das erfindungsgemäße Verfahren kann bei Temperaturen von 60-320°C, bevorzugt bei Temperaturen von 120-250°C und besonders bevorzugt bei Temperaturen von 140-240°C, in der Kolonne durchgeführt werden. Ein in bevorzugter Weise anzulegender Temperaturgradient liegt im angegebenen Temperaturbereich und steigt vom Kolonnenkopf in Richtung Kolonnenfuß. Dabei muß gewährleistet sein, daß die Reaktionstemperatur im Umesterungsbereich nicht oberhalb der Verdamptungstemperatur des eingesetzten Phenols liegt. Es ist deshalb von Vorteil, die erfindungsgemäße Umesterung nicht nur bei Normaldruck, sondern auch bei erhöhtem oder erniedrigtem Druck von 50 mbar bis zu 20 bar durchzuführen. Ein bevorzugter Druckbereich liegt zwischen 0,8 und 15 bar, ein besonders bevorzugter Druckbereich liegt zwischen 1 und 10 bar.

Die Raum-Zeit-Belastung der Kolonne liegt bei 0,05-10 g Gesamtmenge der Reaktionsteilnehmer pro ml wirksames Kolonnenvolumen pro Stunde, bevorzugt bei 0,1-5 g/ml/h, besonders bevorzugt bei 0,2-3 g/ml/h; das wirksame Kolonnenvolumen ist hierbei das der Füllkörperpackung oder das Volumen, in welchem sich feste Einbauten befinden.

Katalysatoren, die für das erfindungsgemäße Verfahren in Frage kommen und die für alle Phasen des erfindungsgemäßen Verfahrens gleich sein könnnen, sind in der Literatur bekannt. Solche Katalysatoren sind beispielsweise Hydride, Oxide, Hydroxide, Alkoholate, Amide oder Salze von (Erd)Alkalimetallen, wie Lithium, Natrium, Kalium, Rubidium, Cäsium, Magnesium und Calcium, bevorzugt von Lithium, Natrium, Kalium, Magnesium und Calcium, besonders

bevorzugt von Lithium, Natrium und Kalium (US-3 642 858, US-3 803 201, EP 1082). Für den Fall des Einsatzes der Alkoholate können diese erfindungsgemäß auch in situ durch Einsatz der elementaren Alkalimetalle und dem erfindungsgemäßen umzusetzenden Alkohol gebildet werden. Salze der (Erd)Alkalimetalle können solche von organischen oder anorganischen Säuren sein, wie von Essigsäure, Propionsäure, Buttersäure, Benzoessäure, Stearinsäure, Kohlensäure (Carbonate oder Hydrogencarbonate), von Salzsäure, Bromwasserstoff- oder Iodwasserstoffsäure, Salpetersäure, Schwefelsäure, Fluorwasserstoffsäure, Phosphorsäure, Blausäure, Rhodanwasserstoff, Borsäure, Zinnsäure, $C_1$-$C_4$-Stannonsäuren oder Antimonsäuren. In bevorzugter Weise kommen als Verbindungen der (Erd)Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Solche (Erd)Alkalimetallverbindungen (gegebenenfalls in situ gebildet aus den freien Alkalimetallen) werden in Mengen von 0,001 bis 2 Gew.-%, bevorzugt 0,005 bis 0,9 Gew.-%, besonders bevorzugt 0,01 bis 0,5 Gew.-%, bezogen auf das umzusetzende Reaktionsgemisch, eingesetzt.

Weitere erfindungsgemäß einsetzbare Katalysatoren sind Lewis-saure Metallverbindungen wie $AlX_3$, $TiX_3$, $UX_4$, $TiX_4$, $VOX_3$, $VX_5$, $ZnX_2$, $FeX_3$ und $SnX_4$, worin X für Halogen, Acetoxy, Alkoxy oder Aryloxy steht (DE-OS 2 528 412, 2 552 907), beispielsweise Titantetrachlorid, Titantetraphenoxid, Titantetraethoxid, Titantetraisopropylat, Titantetradodecylat, Zinntetraisooctylat und Aluminiumtriisopropylat, weiterhin zinnorganische Verbindungen der allgemeinen Formel $(R^4)_{4-x}$-$Sn(Y)_x$, in der Y für einen Rest $OCOR^5$, OH oder OR steht, wobei $R^5$ $C_1$-$C_{12}$-Alkyl, $C_6$-$C_{12}$-Aryl oder $C_7$-$C_{13}$-Alkylaryl bedeutet und $R^4$unabhängig von $R^5$ den Bedeutungsumfang von $R^5$ annehmen kann und x eine ganze Zahl von 1 bis 3 bedeutet, Dialkylzinnverbindungen mit 1-12 C-Atomen im Alkylrest oder Bis(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethyldistannoxan, Hexabutyldistannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew.-% (EP 0 000 879, 0 000 880, 0 039 452, DE-OS 3 445 555, JP 79/63 023), polymere Zinnverbindungen der Formel -[-$R^4$,$R^5$Sn-O-]-, beispielsweise Poly[oxy(dibutylstannylen)], Poly[oxy(dioctylstannylen)], Poly[oxy(butylphenylstannylen)] und Poly[oxy(diphenylstannylen)] (DE-OS 3 445 552), polymere Hydroxystannoxane der Formel -[-$R^4$Sn(OH)-O-]-, beispielsweise Poly(ethylhydroxystannoxan), Poly(butylhydroxystannoxan), Poly(octylhydroxystannoxan), Poly(undecylhydroxystannoxan) und Poly(dodecylhydroxystannoxan) in Mengen von 0,001 bis 20 Gew.-%, bevorzugt von 0,005 bis 5 Gew.-%, bezogen auf Kohlensäurediester (DE 40 06 520). Weitere erfindungsgemäß einsetzbare Zinnverbindungen sind Sn(II)oxid oder besitzen die Formel

$$X^1\text{-}Sn(R^4)_2\text{-}O\text{-}Sn(R^4)_2\text{-}X^2 \qquad \text{(IX)},$$

worin

| | |
|---|---|
| $X^1$ und $X^2$ | unabhängig voneinander OH, SCN, $OR^4$, $OCOR^4$ oder Halogen und |
| $R^4$ | Alkyl, Aryl bedeutet (EP 0 338 760). |

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise $Pb(OH)_2 \cdot 2PbCO_3$, $Pb(OCO\text{-}CH_3)_2$, $Pb(OCO\text{-}CH_3)_2 \cdot 2LiCl$, $Pb(OCO\text{-}CH_3)_2 \cdot 2PPh_3$ in Mengen von 0,001 bis 1, bevorzugt von 0,005 bis 0,25 mol pro Mol Carbonat (JP 57/176 932, JP 01/093 580), andere Blei(II)- und Blei(IV)-verbindungen, wie PbO, $PbO_2$, Mennige, Plumbite ($PbO^{2-}$) und Plumbate ($PbO^-$) (JP 01/093 560), Eisen(III)acetat (JP 61/172 852), weiterhin Kupfersalze und/oder Metallkomplexe, beispielsweise von Alkali-, Zink-, Titan-und Eisen (JP 89/005 588), Kombinationen aus Lewis-Säuren und Protonensäuren (DE-OS 3 445 553) oder Elementverbindungen von Sc, Cr, Mo, W, Mn, Au, Ga, In, Bi, Te und Lanthaniden (EP 0 338 760) in Frage.

Weiterhin sind in den erfindungsgemäßen Verfahren heterogene Katalysatorsysteme einsetzbar. Solche sind beispielsweise Mischoxide aus Silicium und Titan, die durch gemeinsame Hydrolyse von Silicium- und Titanhalogeniden herstellbar sind (JP 54/125 617) und Titandioxide mit hoher BET-Oberfläche >20 $m^2/g$ (DE-OS 40 36 594).

Bevorzugt im erfindungsgemäßen Verfahren einsetzbare Katalysatoren sind Zinn-, Titan- und Zirkoniumverbindungen und die obengenannten Alkali- und Erdalkaliverbindungen, besonders bevorzugt einsetzbare Katalysatoren sind Organozinnverbindungen und Titantetraalkoholate und -phenolate.

Die einzusetzenden Katalysatormengen betragen 0,01 bis 10 mol-%, bevorzugt 0,05 bis 5 mol-% und besonders bevorzugt 0,01 bis 2 mol-%, bezogen auf eingesetzte Phenol- oder Alkylarylcarbonatkomponente und können sich teilweise von den in der Literatur genannten Mengen unterscheiden.

Die folgenden Beispiele soll die vorliegende Erfindung konkret erläutern, wobei sie nicht auf diese Beispiele beschränkt sein soll.

Beispiel 1

In eine mit V4A-Maschendrahtringen (3 x 3 mm) gefüllte, isotherm auf 180°C thermostatisierte Kolonne von 185 cm Länge und 28 mm Durchmesser dosierte man am Kopf kontinuierlich ein auf 160°C vorgeheiztes flüssiges Gemisch aus 150 g/h Phenol und 0,5 mol-% Poly[oxy(butylhydroxystannylen)].

$$\left( -\!\left[ \text{BuSn(OH)-O} \right]_n\!- \right)$$

Diesem Flüssigkeitsstrom schickte man 150 g/h gasförmiges Dimethylcarbonat entgegen, das in einem separaten Apparat verdampft und 35 cm oberhalb des Kolonnenfußes in die Kolonne eingespeist wurde. Am oberen Ende der Kolonne, die ein kurzes Verstärkerteil besaß (15 cm adiabatische Kolonne mit Rücklaufteiler) entnahm man kontinuierlich ein Gemisch aus Methanol und Dimethylcarbonat (Kopfprodukt), am Fuß der Kolonne, die ein kurzes Abtriebsteil besaß (35 cm ölbeheizter Rohrwendelverdampfer), 162 g/h eines Gemisches aus 15,1 Gew.-% Methylphenylcarbonat, 1,6 Gew.-% Diphenylcarbonat, 83,3 Gew.-% Phenol und dem Katalysator (Sumpfprodukt). Das innerhalb von 4,5 h aufgefangene Sumpfprodukt wurde zwischengelagert und nach Beendigung des ersten Durchlaufs erneut am Kopf der Kolonne mit derselben Raum-Zeit-Belastung eingespeist, und diesem unverändert wie im ersten Durchlauf gasförmiges Dimethylcarbonat entgegengeschickt. Am Kolonnenfuß entnahin man nun 173 g/h eines Gemisches aus 26,3 Gew.-% Methylphenylcarbonat, 7,9 Gew.-% Diphenylcarbonat und 65,8 Gew.-% Phenol zusammen mit dem Katalysator (zweiter Durchlauf). Bei einem dritten Durchlauf des aus dem zweiten Durchlauf gewonnenen Produkts unter Dimethylcarbonat-Gegenstrom erhielt man 180 g/h eines Sumpfprodukts der Zusammensetzung 28,6 Gew.-% Methylphenylcarbonat, 10,5 Gew.-% Diphenylcarbonat und 60,8 Gew.-% Phenol. Das entspricht einer Raum-Zeit-Ausbeute für die Methylphenyl- und Diphenylcarbonatbildung, bezogen auf drei Kolonnendurchgänge, von 0,021 kg $1^{-1}$ $h^{-1}$. Wenn man dieses Gemisch nun ohne Dimethylcarbonat-Gegenstrom ein viertes Mal am Kopf der Kolonne einspeiste, erhielt man 196 g/h eines Sumpfprodukts der Zusammensetzung 8,8 Gew.-% Methylphenylcarbonat, 34,7 Gew.-% Diphenylcarbonat und 56,4 Gew.-% Phenol, woraus sich eine Raum-Zeit-Ausbeute für die Bildung von Methylphenyl- und Diphenylcarbonat, bezogen auf 4 Kolonnendurchgänge, von 0,020 kg $1^{-1}$ $h^{-1}$ ergibt.

Beispiel 2

Es wurde die gleiche Verfahrensweise wie im Beispiel 1 gewählt. Als Reaktor wurde aber eine 20-bödige Glockenbodenkolonne von 2 m Lange und einem Durchmesser von 5 cm verwendet. Am Kopf der Kolonne wurden 500 g/h Phenol und 0,5 mol-% Poly[oxy(butylhydroxystannylen)] dosiert, am Fuß der Kolonne 500 g/h gasförmiges Dimethylcarbonat. Nach einem Durchlauf durch die Kolonne erhielt man 554 g/h eines Sumpfprodukts der Zusammensetzung 23,2 Gew.-% Methylphenylcarbonat, 2,1 Gew.-% Diphenylcarbonat und 74,7 Gew.-% Phenol, das noch den Katalysator enthielt. Nach einem zweiten Durchlauf dieses Produkts in der in Beispiel 1 beschriebenen Weise erhielt man 615 g/h eines Sumpfprodukts der Zusammensetzung 36,8 Gew.-% Methylphenylcarbonat, 9,1 Gew.-% Diphenylcarbonat und 54,1 Gew.-% Phenol. Nach einem dritten Kolonnendurchlauf erhielt man 670 g/h eines Sumpfprodukts der Zusammensetzung 45,5 Gew.-% Methylphenylcarbonat, 14,3 Gew.-% Diphenylcarbonat und 40,2 Gew.-% Phenol. Das entspricht einer Raum-Zeit-Ausbeute für die Methylphenyl- und Diphenylcarbonatbildung, bezogen auf drei Kolonnendurchgänge, von 0,034 kg $1^{-1}$ $h^{-1}$. Nach dem vierten Durchlauf des Sumpfproduktes, wobei kein Dimethylcarbonat im Gegenstrom geführt wurde, hatte das Sumpfprodukt die Zusammensetzung 13,5 Gew.-% Methylphenylcarbonat, 50,7 Gew.-% Diphenylcarbonat und 35,8 Gew.-% Phenol, woraus sich eine Raum-Zeit-Ausbeute für die Bildung von Methylphenyl- und Diphenylcarbonat, bezogen auf 4 Kolonnendurchläufe, von 0,032 g $1^{-1}$ $h^{-1}$ ergibt. Der Katalysator wurde auch hier in keinem Fall entfernt, sondern in allen Durchläufen mit dem Sumpfprodukt zurückgeführt. Die Sumpfzusammensetzungen wurden durch gaschromatographische Analyse (GC) als Gew.-%-Zusammensetzungen bestimmt.

Aus den beschriebenen Versuchen sieht man, daß mit dem erfindungsgemäßen Verfahren hohe Phenolumsätze, realisierbar sind, bzw. Produkte mit niedrigen Phenol- und hohen Umesterungsproduktgehalten erreicht werden. So beträgt der Anteil an Umesterungsprodukten im Endprodukt des Beispiels 2 ca. 65 Gew.-%. Das ist erheblich mehr, als in WO 91/09832 gezeigt wird, wo nur ca. 16 Gew.-% an Umesterungsprodukten erhalten werden. Diese sehr guten Umsätze werden bei gleichen oder besseren Gesamtraum-Zeit-Ausbeuten erhalten: In den obigen Versuchen wurden Raum-Zeit-Ausbeuten von 0,02 kg $1^{-1}$ $h^{-1}$, bzw. 0,032 kg $1^{-1}$ $h^{-1}$, bezogen auf die Summe aus Methylphenyl- und Diphenylcarbonat über 4 Kolonnendurchgänge, erreicht im Vergleich zu Raum-Zeit-Ausbeuten von 0,018, bzw. von 0,015 kg $1^{-1}$ $h^{-1}$, wie in WO 91/09832 beschrieben. Das bedeutet, daß erfindungsgemäß mit nur einer Kolonne, die ein vergleichbares Reaktionsvolumen wie die Apparatur in WO 91/09832 besitzt, pro Zeiteinheit mindestens gleiche Mengen an Arylcarbonaten hergestellt werden können.

**Patentansprüche**

1. Verfahren zur Herstellung eines organischen Carbonats mit mindestens einer aromatischen Estergruppe der Formel

$$R^1\text{-OCOO-}R^2,$$

in der

$R^2$ Phenyl oder Naphthyl sowie ein- bis dreifach durch geradkettiges oder verzweigtes $C_1$-$C_4$-Alkyl, geradkettiges oder verzweigtes $C_1$-$C_4$-Alkoxy, Cyano und/oder Halogen substituiertes Phenyl bzw. Naphthyl bedeutet und
$R^1$ unabhängig von $R^2$ den Bedeutungsumfang von $R^2$ annehmen oder geradkettiges oder verzweigtes $C_1$-$C_6$-Alkyl bedeuten kann,

durch katalysierte Umsetzung von je 0,1-10 mol eines organischen Carbonats mit mindestens einer aliphatischen Estergruppe der Formel

$$R^1\text{-OCOO-}R^3,$$

in der

$R^3$ geradkettiges oder verzweigtes $C_1$-$C_6$-Alkyl bedeutet und
$R^1$ den obigen Bedeutungsumfang hat,

mit je 1 mol einer phenolischen Verbindung der Formel

$$R^2\text{-OX},$$

in der

$R^2$ den obigen Bedeutungsumfang hat und
X für Wasserstoff oder für -COO-$C_1$-$C_6$-Alkyl mit geradkettiger oder verzweigter Alkylgruppe steht,

in Gegenwart eines an sich bekannten Umesterungskatalysators bei 60-320°C in einem kolonnenartigen Reaktor, wobei das mindestens eine aromatische Estergruppe enthaltende organische Carbonat im unteren Teil der Kolonne und die als Reaktionsprodukt mitentstehende alkoholische Verbindung der Formel

$$R^3\text{-OX},$$

in der

X und $R^3$ die genannte Bedeutung haben,

im oberen Teil der Kolonne entnommen werden, dadurch gekennzeichnet, daß das im unteren Teil der Kolonne flüssig entnommene Sumpfprodukt, das mindestens eine aromatische Gruppe enthaltendes Carbonat, noch nicht umgesetztes Phenol und gegebenenfalls geringe Mengen des mindestens eine aliphatische Estergruppe enthaltenden Carbonats enthält, 1 bis 10 weiteren Durchläufen durch denselben Reaktor unter Zwischenlagerung des Sumpfprodukts unterworfen wird, wobei bei den zuletzt angesetzten 1-4 Durchläufen der weitere Zusatz des organischen Carbonats mit mindestens einer aliphatischen Estergruppe entfallen kann.

2. Verfahren nach Anspruch 1, dadurch gekennzeichnet, daß eine phenolische Verbindung der Formel

$$R^{12}\text{-OH}$$

eingesetzt wird, in der

$R^{12}$ Phenyl oder einfach durch $C_1$-$C_4$-Alkyl, $C_1$-$C_4$-Alkoxy oder Chlor substituiertes Phenyl bedeutet.

**3.** Verfahren nach Anspruch 1, dadurch gekennzeichnet, daß Dimethylcarbonat und Phenol zu Methylphenylcarbonat und Diphenylcarbonat umgesetzt werden.

**4.** Verfahren nach Anspruch 1, dadurch gekennzeichnet, daß symmetrische Dialkylcarbonate der Formel

$$R^3\text{-OCOO-}R^3$$

eingesetzt werden, in der

$R^3$ geradkettiges oder verzweigtes $C_1$-$C_6$-Alkyl bedeutet.

## Claims

**1.** Process for the preparation of an organic carbonate having at least one aromatic ester group of the formula

$$R^1\text{-OCOO-}R^2,$$

in which

$R^2$ denotes phenyl or naphthyl or phenyl or naphthyl each of which is mono- to trisubstituted by straight-chain or branched $C_1$-$C_4$-alkyl, straight-chain or branched $C_1$-$C_4$-alkoxy, cyano and/or halogen and

$R^1$ can, independently of $R^2$, assume the range of meanings of $R^2$ or can denote straight-chain or branched $C_1$-$C_6$-alkyl,

by catalysed reaction of 0.1-10 mol in each case of an organic carbonate having at least one aliphatic ester group of the formula

$$R^1\text{-OCOO-}R^3,$$

in which

$R^3$ denotes straight-chain or branched $C_1$-$C_6$-alkyl and
$R^1$ has the above range of meanings,

with 1 mol in each case of a phenolic compound of the formula

$$R^2\text{-OX},$$

in which

$R^2$ has the above range of meanings and

X represents hydrogen or -COO-$C_1$-$C_6$-alkyl having a straight-chain or branched alkyl group,

in the presence of a transesterification catalyst known per se at 60-320°C in a column-type reactor, the organic carbonate containing at least one aromatic ester group being withdrawn from the bottom part of the column and the alcoholic compound co-formed as a reaction product of the formula

$$R^3\text{-OX},$$

in which

X and $R^3$ have the meaning mentioned,

being withdrawn from the top part of the column, characterised in that the bottom product withdrawn in the liquid state from the bottom part of the column which contains a carbonate containing at least one aromatic ester group,

still unreacted phenol and, possibly, small amounts of the carbonate containing at least one aliphatic ester group, is subjected to 1 to 10 further passes through the same reactor with intermediate storage of the bottom product, where the further addition of the organic carbonate having at least one aliphatic ester group can be dispensed with in the last 1-4 passes employed.

**2.** Process according to Claim 1, characterised in that a phenolic compound of the formula

$$R^{12}\text{-OH}$$

is used in which

$R^{12}$ denotes phenyl or phenyl monosubstituted by $C_1$-$C_4$-alkyl, $C_1$-$C_4$-alkoxy or chlorine.

**3.** Process according to Claim 1, characterised in that dimethyl carbonate and phenol are reacted to form methyl phenyl carbonate and diphenyl carbonate.

**4.** Process according to Claim 1, characterised in that symmetrical dialkyl carbonates of the formula

$$R^3\text{-OCOO-}R^3$$

are used, in which

$R^3$ denotes straight-chain or branched $C_1$-$C_6$-alkyl.

## Revendications

**1.** Procédé pour la préparation d'un carbonate organique présentant au moins un groupe ester aromatique de la formule :

$$R^1\text{-OCOO-}R^2,$$

dans laquelle
$R^2$ est un groupe phényle ou naphtyle ainsi qu'un groupe phényle ou naphtyle une à trois fois substitué par un groupe alkyle en $C_1$-$C_4$ linéaire ou ramifié, un groupe alcoxy en $C_1$-$C_4$ linéaire ou ramifié, un groupe cyano et/ou un halogène et
$R^1$ peut prendre indépendamment de $R^2$ l'étendue de $R^2$ ou peut-être un groupe alkyle en $C_1$-$C_6$ linéaire ou ramifié, par réaction catalysée à chaque fois de 0,1-10 mol d'un carbonate organique présentant au moins un groupe ester aliphatique de la formule :

$$R^1\text{-OCOO-}R^3,$$

dans laquelle
$R^3$ est un groupe alkyle en $C_1$-$C_6$ linéaire ou ramifié et
$R^1$ a l'étendue ci-dessus,
avec à chaque fois 1 mol d'un composé phénolique de la formule :

$$R^2\text{-OX},$$

dans laquelle
$R^2$ a l'étendue ci-dessus et
X est l'hydrogène ou un groupe -COO- alkyle en $C_1$-$C_6$ présentant un groupe alkyle linéaire ou ramifié,
en présence d'un catalyseur de transestérification connu en lui-même à une température de 60-320°C dans un réacteur de type colonne, le carbonate organique contenant au moins un groupe ester aromatique étant prélevé dans la partie inférieure de la colonne et le composé alcoolique produit simultanément en tant que produit de réaction de la formule :

$$R^3\text{-OX},$$

dans laquelle
X et $R^3$ ont la signification citée,
dans la partie supérieure de la colonne, caractérisé en ce que le produit de queue prélevé sous forme liquide dans la partie inférieure de la colonne, qui contient du carbonate contenant au moins un groupe ester aromatique, du phénol n'ayant pas encore réagi et éventuellement de faibles quantités du carbonate contenant au moins un groupe ester aliphatique, est soumis à de 1 à 10 passages supplémentaires à travers le même réacteur avec stockage intermédiaire du produit de queue, l'addition supplémentaire du carbonate organique présentant au moins un groupe ester aliphatique pouvant être supprimée pour les 1-4 derniers passages réalisés.

**2.** Procédé selon la revendication 1, caractérisé en ce qu'on utilise un composé phénolique de la formule :

$$R^{12}\text{-OH},$$

dans laquelle
$R^{12}$ est un groupe phényle ou un groupe phényle une fois substitué par un groupe alkyle en $C_1$-$C_4$, un groupe alcoxy en $C_1$-$C_4$ ou par le chlore.

**3.** Procédé selon la revendication 1, caractérisé en ce qu'on transforme du carbonate de diméthyle et du phénol en carbonate de méthyle et de phényle et en carbonate de diphényle.

**4.** Procédé selon la revendication 1, caractérisé en ce qu'on utilise des carbonates de dialkyles symétriques de la formule :

$$R^3\text{-OCOO-}R^3,$$

dans laquelle
$R^3$ est un groupe alkyle en $C_1$-$C_6$ linéaire ou ramifié.

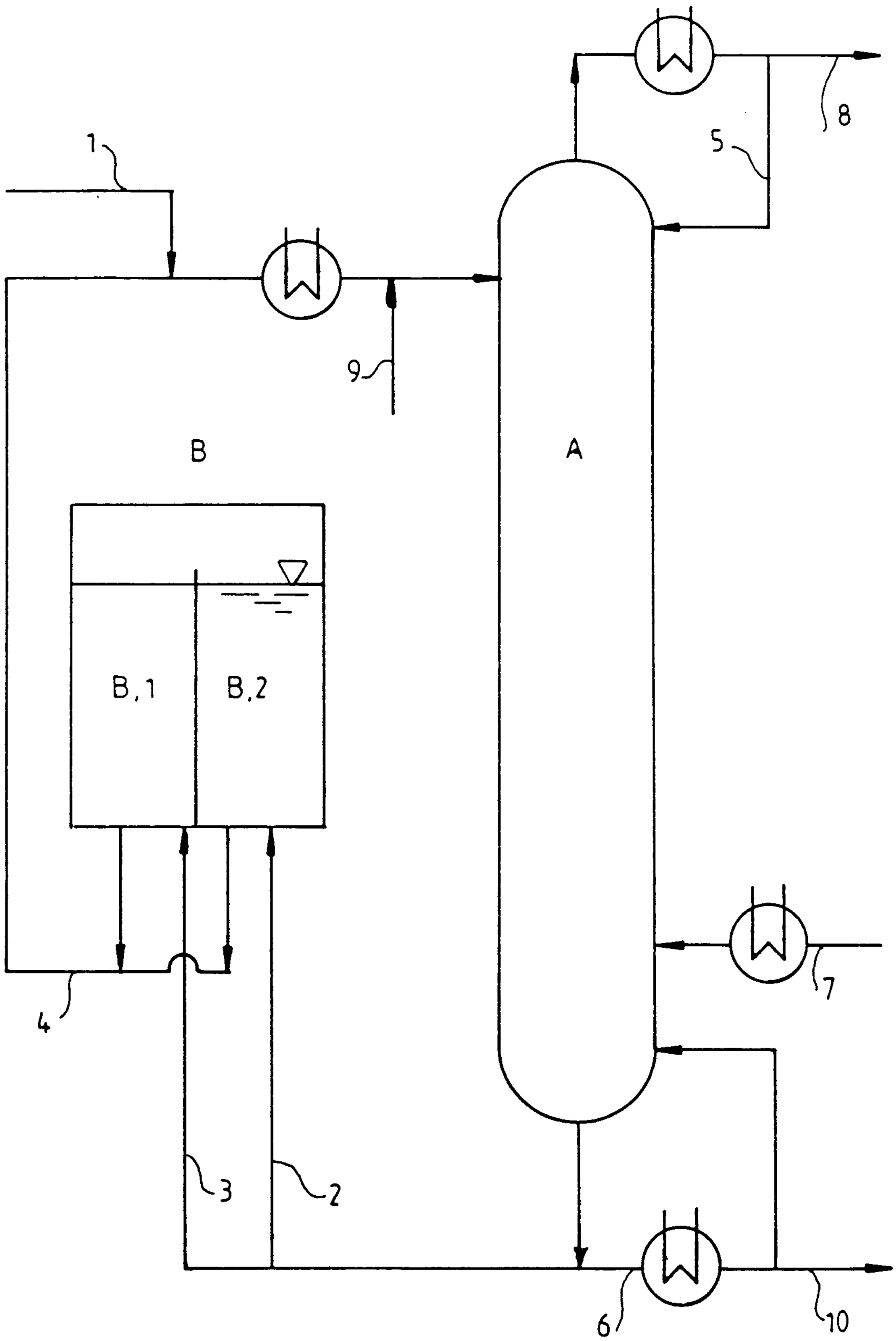


FIG.1